**Europäisches Patentamt**

(19) **European Patent Office**

**Office européen des brevets**

(11) Numéro de publication: **0 117 962 B1**

(12) # FASCICULE DE BREVET EUROPEEN

(45) Date de publication du fascicule du brevet:
**16.09.87**

(51) Int. Cl.⁴: **C 11 C 3/00, A 61 K 37/22**

(21) Numéro de dépôt: **83402558.7**

(22) Date de dépôt: **29.12.83**

(54) **Procédé de préparation de graisses peroxydées par transformation biologique à faible énergie, produits obtenus par ce procédé, mélanges de ces produits avec des additifs et nouveaux médicaments constitués de ces produits.**

(30) Priorité: **06.01.83 FR 8300152**
**17.11.83 FR 8318304**

(43) Date de publication de la demande:
**12.09.84 Bulletin 84/37**

(45) Mention de la délivrance du brevet:
**16.09.87 Bulletin 87/38**

(84) Etats contractants désignés:
**AT BE CH DE FR GB IT LI LU NL SE**

(56) Documents cités:
**EP - A - 0 031 113**
**FR - A - 2 461 744**

(73) Titulaire: **Lepeltier, Marie-Françoise, 7 rue des Bergères Bâtiment Prairial B, F-91440 les Ulis (FR)**
Titulaire: **Le Bihan, Yvon, 2 rue Toul al Laer, F-29000 Quimper (FR)**
Titulaire: **Beaufume, Nicole, 7 rue des Bergères Bâtiment Prairial B, F-941440 les Ulis (FR)**
Titulaire: **Le Bihan, Lucienne, 2 rue Toul al Laer, F-29000 Quimper (FR)**
Titulaire: **Etienne, Nicole, 27 rue Ducouedic, F-75014 Paris (FR)**

(72) Inventeur: **Lepeltier, Marie-Françoise, 7, rue des Bergères Bâtiment Prairial B, F-91440 Les Ulis (FR)**
Inventeur: **Le Bihan, Yvon, 2, rue Toul Al Lear, F-29000 Quimper (FR)**
Inventeur: **Laidet Maurice, Route de Proquiès Frejairolles, 81000 Albi (FR)**

(74) Mandataire: **Hasenrader, Hubert et al, Cabinet BEAU DE LOMENIE 55, rue d'Amsterdam, F-75008 Paris (FR)**

**Description**

La présente invention concerne un procédé de préparation de graisses peroxydées, à partir de corps gras, d'origine végétale, animale ou minérale et l'application industrielle et thérapeutique des produits ainsi préparés.

On connaît déjà un procédé de préparation d'émulsions huileuses, qui consiste à peroxyder des corps gras tels que les huiles d'arachide, d'olive, de sésame, de colza, huile de pied de bœuf, huile de lard. Puis ces corps gras peroxydés sont mis en présence d'eau alcaline, à un pH égal ou supérieur à 8 et ils se mettent en émulsion spontanément, sans adjonction d'émulsifiant. La peroxydation se fait, par apport d'oxygène ou d'eau oxygénée, à une température supérieure à 20°C. Les émulsions ainsi obtenues sont utilisées notamment pour l'abattage de poussières, pour le lavage de moules de fonderie, dans l'industrie de la laine et du cuir.

Cependant, de telles émulsions, bien que donnant de bons résultats, ont dues être améliorées et on a cherché à augmenter leur action dans de nombreux domaines d'applications, industriels ou bien diététiques, cosmétologiques ou bien agro-alimentaires.

On connait la peroxydation de matières grasses en traitant des graisses insaturées sous forme liquide. On les soumet à une température inférieure à 100°C et on ajoute de l'oxygène et de la chlorophyle en présence de lumière exempte de rayons ultra-violets. Un tel procédé est décrit dans le brevet américain n° 2 727 857.

Le brevet français n° 1 374 348 décrit la peroxydation de corps gras tels que l'huile d'olive, d'arachide, d'oeillette, de maïs, de moutarde par traitement avec de l'oxygène, en absence de lumière à une température supérieure à 20°C. On obtient alors des peroxydes de corps gras dont l'indice d'iode est compris entre environ 50 et 150 que l'on peut mettre en émulsion aqueuse en milieu de pH égal ou supérieur à 8.

Ces matières grasses peuvent trouver une application thérapeutique, telle que le traitement de dermatoses et lèpre. Ces applications thérapeutiques sont décrites dans le brevet spécial de médicament n° 2 330 M.

Enfin, le brevet français n° 2 461 744 décrit une peroxydation de matières grasses par soufflage d'air, la température des matières grasses étant comprise entre 75 et 100°C, en présence de lumière ultraviolette de longueur d'onde 184,9 à 253,7 nm (1 849 à 2 537 Å). Ces matières grasses ainsi peroxydées ont des propriétés thérapeutiques permettant le traitement des rhumatismes notamment. Or, le traitement par des rayons ultra-violets artificiels, à des puissances de 5 à 20 Watts par litre de matière traitée, constitue une condition traumatisante pour le produit traité. Ainsi, les produits biologiques éventuellement contenus dans les matières grasses sont détruits ou au moins très modifiés de façon indésirable, par l'élévation de température jusqu'à 100°C, par la rapidité du chauffage et par le bombardement par des rayons U.V. non naturels. Par exemple, si on traite par ce procédé des corps gras provenant de matière vivante, contenant donc des enzymes, protides, protéines, etc., ceux-ci sont détruits.

Un but de l'invention est donc de traiter des matières grasses, notamment d'origine animale, sans que les constituants biologiques soient détruits.

L'invention concerne donc un procédé de peroxydation de corps gras par transformation à faible énergie permettant d'obtenir des produits donnant de meilleurs résultats et permettant des innovations au niveau des applications dans les domaines précités.

Le procédé selon l'invention est une peroxydation de matières grasses par transformation biologique à faible énergie, à savoir par introduction en continu d'un mélange gazeux oxygéné dans le corps gras, de préférence sous forme liquide, en faisant varier par paliers irréguliers la température du corps gras, la température étant maintenue sensiblement constante pendant des intervalles variables de temps déterminés. La réaction se fait en présence de lumière naturelle ou électrique à faible énergie et spectre étendu et la durée totale du traitement peut être de 7 à 28 jours et plus.

Les durées des phases de traitement pendant lesquelles la température reste sensiblement constante peuvent être considérées comme des cycles de n×7 heures ±1. A titre indicatif, en ce qui concerne certains modes opératoires, ces durées peuvent être comprises entre 34 et 49 heures environ.

Les durées pendant lesquelles la température reste sensiblement constante sont de préférence égales à 41 h, 49 h ou n×7 heures (par exemple: 21×7 = 147 heures), n étant un nombre entier.

Selon le procédé de l'invention, le corps gras liquide subit, lors d'un intervalle de chauffage, une modification de coloration, en passant de la couleur jaunâtre initiale à la couleur rouge foncé puis à la couleur blanc luminescent.

Lorsqu'on traite une huile d'arachide, le changement de couleur se fait selon le volume de produit traité, à différentes phases du cycle de traitement. En particulier, pour un volume de 25 l d'huile d'arachide non traitée et non amorcée au préalable. Ce changement de couleur se produit à 74°C pendant la durée de 49 heures, juste avant de redescendre à la température de 64°C.

Lorsqu'on traite une huile de colza, le changement de couleur se fait comme ci-dessus en fonction du volume et du traitement préalable pendant la phase de traitement à 58°C environ pendant une durée de 42 h.

Lorsqu'on traite une huile d'olive vierge, le changement de couleur se fait (en fonction du volume et du traitement) pendant la phase de traitement à 64°C environ, d'une durée de 41 h environ.

De préférence, le mélange gazeux est de l'air, notamment pour le traitement de l'huile de colza et de l'huile d'arachide par exemple.

Le mélange gazeux peut être de l'oxygène, par exemple pour le traitement de l'huile d'olive vierge.

Eventuellement on peut induire la transformation soit par introduction d'une quantité de produit fini, qui a la fonction d'un germe ou d'une mère, soit par oxygénation et éclairage préalables du produit à traiter, soit par les deux modes d'induction conjugués. La durée pour obtenir le produit fini est réduite en fonction des paramètres d'induction.

On peut utiliser des montages électromagnétiques optiques et acoustiques permettant soit de transformer les paliers et cycles de traitement des modes opératoires, soit de dynamiser les processus bio-énergétiques de transformations de structure des produits traités. (Générateurs ultrasoniques, systèmes holographiques, machine de Sprink (perturbations du champ électromagnétique et du vecteur gravitationnel)).

Or, selon la présente invention, on a trouvé de manière surprenante que les corps gras ayant subi un tel traitement de peroxydation par transformation biologique à faible énergie, ont des propriétés thérapeutiques remarquables.

On a trouvé notamment que ces corps gras peroxydés par transformation biologique à faible énergie sont des produits cicatrisants antalgiques, anti-inflammatoires et bactériostatiques. Par ailleurs, ce sont des facteurs de croissance pour les animaux.

La description suivante, en regard des dessins annexés à titre d'exemples non limitatifs, permettra de comprendre comment l'invention peut être mise en pratique.

La fig. 1 est un schéma du mode opératoire de traitement de l'huile végétale d'arachide.

La fig. 2 est un schéma du mode opératoire de traitement de l'huile de colza.

La fig. 3 est un schéma du mode opératoire de traitement de l'huile d'olive vierge.

La fig. 4 montre une courbe de croissance obtenue par administration du corps gras selon l'invention à des lapins.

Selon l'invention, on peut traiter toutes sortes de corps gras, par exemple l'huile d'arachide, de colza, d'olive vierge ou des matières grasses animales, par exemple un mélange de moëlle de taureau et d'huile végétale traitée selon le procédé de l'invention ou bien par exemple, un mélange d'embryons d'animaux ou de végétaux ou bien d'organites cellulaires ou bien d'oligo-éléments, ou bien de vitamines, avec une huile traitée selon le procédé de l'invention.

On peut effectuer plusieurs phases de traitement qui peuvent être espacées par des phases de repos, c'est-à-dire des périodes de temps pendant lesquelles on maintient le mélange réactionnel à température ambiante, ou température voisine de 0°C.

Par ailleurs, on peut effectuer plusieurs phases de traitement qui peuvent être espacés par des phases dites «phase de pont modulant» suivant un intervalle de chauffage pendant lequel le mélange réactionnel subit une modification de coloration, en passant de la couleur jaunâtre initiale à la couleur rouge foncé puis à la couleur blanc luminescent. Comme précisé ci-avant, ces phases de coloration se situent à des instants différents du mode opératoire suivant le volume de produit traité et suivant les variations de certains paramètres de notre structure de l'espace et du temps, à savoir par exemple variations de biorythmes cosmiques et suivant les paramètres inducteurs mentionnés ci-dessus.

Plus particulièrement, les durées des phases de traitement, pendant lesquelles la température reste sensiblement constante peuvent être considérées comme des cycles de $n \times 7$ heures $\pm 1$ (n étant un nombre entier). A titre indicatif, en ce qui concerne certains modes opératoires, ces durées peuvent être comprises entre 34 et 49 heures environ. Les durées des phases de traitement sont de préférence égaux à 34 h, 41 h, 49 h, ou bien $n \times 7$ heures $\pm 1$ environ, à savoir sont des multiples de 7 approximativement. ($5 \times 7 = 35$ h, $6 \times 7 = 42$ h, $7 \times 7 = 49$ h, $21 \times 7 = 147$ h...)

Les phases de pont modulant sont par exemple de 70 h ($10 \times 7$ h) ou de 42 h environ ($7 \times 6$ h).

Les phases de repos pendant lesquelles la température est maintenue à température ambiante ou voisine de 0°C sont environ de 147 h, à savoir un multiple de 7 h ($21 \times 7$ h).

Ainsi, les cycles de traitement selon l'invention se décomposent comme suit:

– plusieurs phases de traitement pendant chacune desquelles la température est maintenue constante et de durées sensiblement égales à un multiple de 7 h ($\pm 1$ h), $t = 7 \times a$, avec a nombre entier compris entre 1 et 10 ($1 \leqslant a \leqslant 10$), chaque phase ayant une température distincte;

– plusieurs phases de «pont modulant» pendant chacune desquelles la température est maintenue constante et de durées sensiblement égales à un multiple de 7 ($\pm 1$ h), $t = 7 \times b$, avec b nombre entier compris entre 6 et 10 de préférence b = 6 et b = 10;

– plusieurs phases de repos pendant chacune desquelles la température est maintenue égale à la température ambiante ou voisine de 0°C et de durée sensiblement égale à un multiple de 7 h ($\pm 1$ h), ($t = 7 \times c$, avec c nombre entier égale de préférence à 21).

En ce qui concerne ces trois phases, les multiples de 7 peuvent varier suivant la structure et le volume de produit à traiter. Les exemples ci-dessus sont donnés pour un volume de 25 l des produits de référence.

Exemple 1

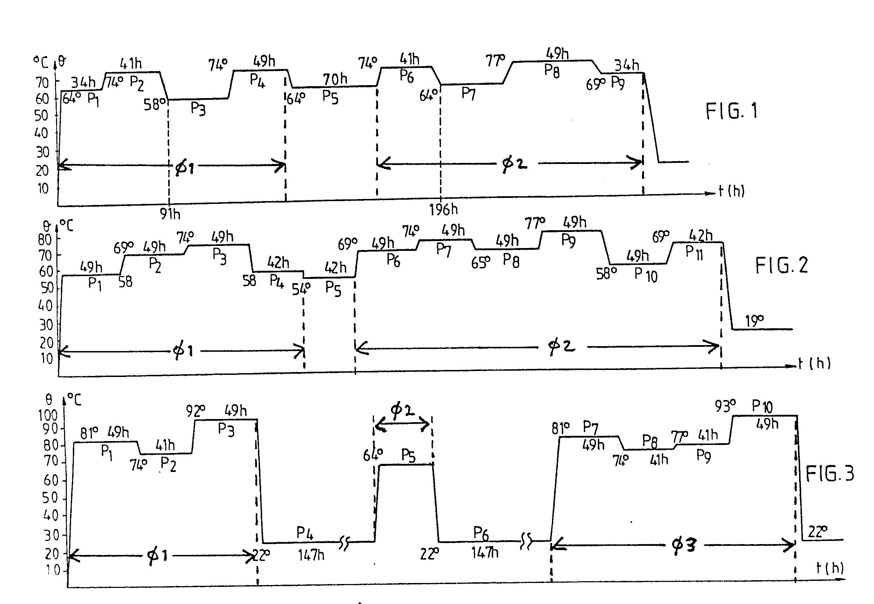

En se référant à la figure 1, on voit que l'étape $\varnothing 1$ initiale de traitement pour l'huile végétale d'arachide comporte quatre phases de traitement $P_1$, $P_2$, $P_3$, $P_4$.

Le mélange gazeux est de l'air.

La phase de pont modulant est la phase $P_5$.

La seconde étape de traitement $\varnothing 2$ après la

phase de pont modulant $P_5$ comporte quatre phases $P_6$, $P_7$, $P_8$, et $P_9$.

Selon un premier mode de réalisation du procédé de traitement de l'huile végétale d'arachide, on procède de la manière suivante:

Etape initiale ∅1:
- On chauffe le mélange réactionnel progressivement en introduisant de l'air, jusqu'à atteindre la température de 64°C.
- On maintient cette température, en introduisant l'air, pendant une durée de 34 heures ($P_1$).
- On hausse la température en continuant d'introduire l'air jusqu'à atteindre 74°C.
- On maintient cette température en aérant pendant une durée de 41 heures ($P_2$).
- On descend la température en continuant d'aérer le mélange réactionnel, jusqu'à atteindre 58°C.
- On maintient cette température en aérant pendant une troisième phase ($P_3$).
- Puis on hausse cette température en aérant toujours jusqu'à 74°C.
- On maintient cette température en aérant pendant une durée de 49 heures ($P_4$).

Durant cette étape, il se produit un double changement de coloration du mélange réactionnel qui passe du jaune très clair au rouge tonique pour revenir au blanc limpide, presque opalescent, et ceci pour un volume de produit traité, V = 25 l environ.

On peut descendre la température jusqu'à 19°C, tout en continuant l'introduction d'air dans le mélange réactionnel (non représenté).

On obtient alors une première qualité d'huile d'arachide peroxydée. Ce produit peut être utilisé tel quel. La conservation de ce produit nécessite un conteneur transparent aux rayons solaires, de préférence le conteneur ne doit pas être bouché hermétiquement si le temps de stockage s'avère devoir être assez long.

Selon un autre mode de réalisation de ce procédé, on effectue l'étape initiale ∅1, que l'on enchaîne à l'étape ∅2 par une phase de «pont modulant».
- Phase de pont modulant – Après $P_4$, on descend la température à 64°C en continuant d'aérer le produit.
- On maintient le produit à cette température pendant une durée de 70 h environ $P_5$).

Seconde étape ∅2:
On chauffe progressivement en aérant jusqu'à atteindre la température de 74°C.
- On maintient cette température en introduisant l'air, pendant une durée de 41 h ($P_6$).
- On descend la température progressivement, en aérant jusqu'à atteindre 64°C.
- On maintient cette température en aérant pendant une septième phase ($P_7$).
- Puis on monte progressivement la température en aérant, jusqu'à atteindre 77°C et on maintient cette température en aérant pendant une durée de 49 h ($P_8$).
- On redescend la température en aérant jusqu'à atteindre 69°C et on maintient cette température pendant une durée de 34 h en aérant ($P_9$).
- Enfin, on cesse de chauffer et on continue d'aérer et d'éclairer le produit jusqu'à ce que la température atteigne 19°C.

Le produit final peut être utilisé notamment comme catalyseur bio-énergétique, activateur d'échanges chimiques, gazeux, bio-électriques. A titre d'exemple, il peut être utilisé pour le traitement des cuirs, des bois, et autres techniques industrielles. En tant que produit de base, il peut entrer dans la constitution de divers produits à usage diététique, cosmétologique, agro-alimentaire.

On peut éventuellement réaliser l'étape ∅1 puis l'étape ∅2 après une période d'attente liée à certains rythmes cosmiques ou on peut réaliser ∅1 et ∅2 en les reliant par la phase de pont modulant.

Exemple 2
Traitement de l'huile de colza (figure 2).
De même que pour l'huile d'arachide, on effectue une première étape ∅1 comportant quatre phases de traitement $P_1$, $P_2$, $P_3$, $P_4$, après laquelle on peut arrêter le traitement et conserver un produit prétraité qui peut être utilisé tel quel ou qui peut subir une seconde étape ∅2 de traitement. Cette étape ∅2 de traitement comporte 6 phases de traitement ($P_6$, $P_7$, $P_8$, $P_9$, $P_{10}$, $P_{11}$).

Le changement de couleur s'effectue lors de la phase de traitement $P_4$, lorsqu'il s'agit de la transformation de 25 l de produit initial, sans initiateur.

Enfin, on peut effectuer une phase de «pont modulant» entre les étapes ∅1 et ∅2.

Les durées et températures de traitement sont les suivantes:

Tableau I

| Phases | Durées Traitement H | Température Traitement °C |
|---|---|---|
| $P_1$ | 49 | 58 |
| $P_2$ | 49 | 69 |
| $P_3$ | 49 | 74 |
| $P_4$ changement couleur (25 1) | 42 | 58 |
| $P_5$ pont modulant | 42 | 54 |
| $P_6$ | 49 | 69 |
| $P_7$ | 49 | 74 |
| $P_8$ | 49 | 69 |
| $P_9$ | 49 | 77 |
| $P_{10}$ | 49 | 58 |
| $P_{11}$ | 42 | 69 |

Puis on arrête le chauffage en continuant d'aérer et d'éclairer le produit jusqu'à atteindre la température de 19°C. On conserve le produit à 19°C environ à l'air libre.

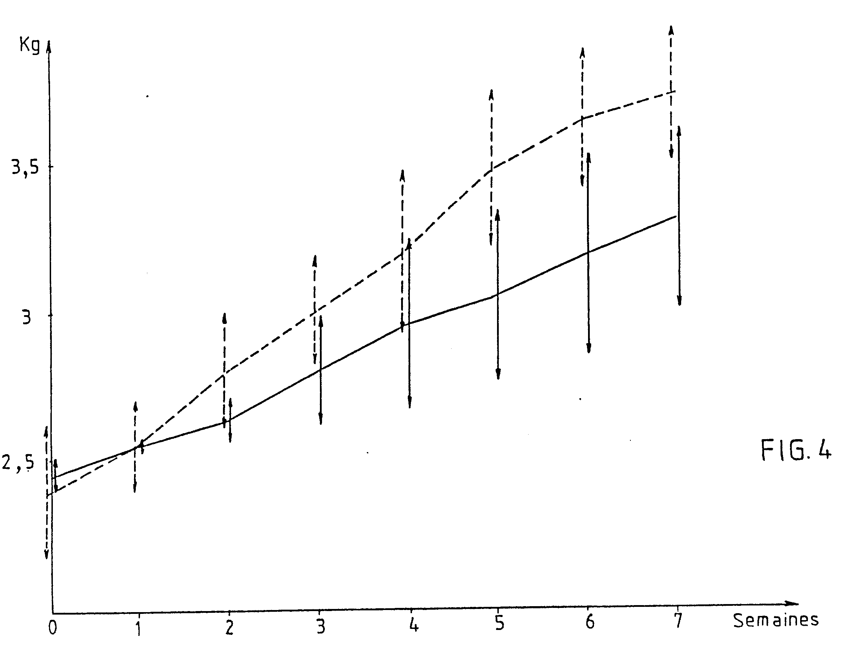

Exemple 3
Traitement de l'huile d'olive vierge (figure 3).
Le gaz de traitement est de l'oxygène.

On effectue trois étapes de traitement séparées par deux phases de repos.

La première étape $\varnothing$1 comporte trois phases ($P_1$, $P_2$ et $P_3$), la deuxième étape $\varnothing$2 comporte une phase $P_5$, la troisième étape $\varnothing$3 comporte quatre phases ($P_7$, $P_8$, $P_9$ et $P_{10}$).

Le changement de coloration du jaune clair au rouge brique puis blanc opalescent s'effectue lors de la phase $P_5$ (étape $\varnothing$2) et plus rarement lors de la phase $P_3$ (étape $\varnothing$1).

Le tableau II donne les durées et les températures.

#### Tableau II

| Phases | Durée Traitement h | Température Traitement °C |
|---|---|---|
| $P_1$ | 49 | 81 |
| $P_2$ | 41 | 74 |
| $P_3$ | 49 | 92 |
| $P_4$ Repos | 147 (21×7) | 22 |
| $P_5$ | – | 64 |
| $P_6$ Repos | 147 (21×7) | 22 |
| $P_7$ | 49 | 81 |
| $P_8$ | 41 | 74 |
| $P_9$ | 41 | 77 |
| $P_{10}$ | 49 | 93 |

Puis on arrête le chauffage en continuant d'aérer à l'oxygène et d'éclairer le produit, jusqu'à atteindre la température de 22°C.

Le produit doit être conservé avec aération et lumière solaire à 22°C.

#### Exemple 4

Traitement d'un mélange de moëlle de taureau fraîche et d'huile d'olive traitée selon l'invention.

On effectue un mélange homogène d'huile d'olive vierge traitée selon l'exemple 3 avec de la moëlle de taureau fraîche.

{ Moëlle de taureau 44%.
{ Huile traitée 56%.

Le traitement comporte deux étapes de traitement 1 et 2 séparées par une phase de repos.

Le mélange gazeux est l'air.

#### Première étape $\varnothing$1

– On chauffe progressivement jusqu'à 47°C en aérant le mélange et on maintient le mélange à cette température en introduisant de l'air, pendant 49 heures (l'opération s'effectue sous éclairage).

– On descend la température à 37°C en continuant d'aérer le mélange réactionnel et on maintient la température pendant une durée de 49 heures.

– On remonte la température à 56°C en aérant le mélange réactionnel et on maintient cette température pendant une durée de 49 heures en aérant.

– On descend la température jusqu'à 15°C en aérant.

On conserve ce mélange au froid dans un réfrigérateur pendant une durée de 37 fois 7 heures (259 h).

#### Deuxième étape $\varnothing$2

Le tableau III donne les températures de traitement et les durées des phases.

L'opération s'effectue en aérant et en éclairant le mélange réactionnel.

#### Tableau III

| Température °C | Durée heure |
|---|---|
| 56 | 49 |
| 37 | 49 |
| 49 | 49 |

Enfin on descend la température à 19°C en continuant d'aérer et d'éclairer le produit.

Le produit obtenu est conservé à température ambiante et à l'air libre.

Les produits obtenus selon les exemples décrits ci-dessus peuvent être utilisés purs ou en mélange. Ils peuvent entrer dans la constitution de produits à usage diététique, cosmétologique, agro-alimentaire. Les produits ainsi obtenus peuvent être électro-vibrés et triturés.

On peut également effectuer des mélanges:

{ d'huile peroxydée
{ d'eau
{ d'essence
{ d'alcool.

Ces mélanges peuvent être selon les pourcentages de produits constitutifs utilisés comme source de nouveau carburant.

Enfin, les produits gras peroxydés selon les principes revendiqués peuvent également servir de base à des mélanges contenant:

– des huiles essentielles,
– des extraits et embryons végétaux,
– des protéines végétales ou animales,
– des organites cellulaires,
– des vitamines,
– des oligo-éléments,
– des minéraux,
– des métaux,
– des solutions aqueuses (par exemple eau $\varphi$, plasma, ainsi qu'à des macérats).

Les mélanges obtenus sont biodynamiques et bioénergétiques.

#### Exemple 5

On utilise 12 lapins néo-zélandais mâles dont 6 traités et 6 témoins, d'un poids voisin de 2,5 kg au début de l'expérimentation, logés dans des cages métalliques grillagées individuelles. On leur donne pendant toute la durée de l'étude un aliment en granulés et de l'eau de boisson renouvelée tous les jours et distribuée en biberons.

Les animaux sont tondus avec une tondeuse électrique sur la région dorsale rétroscapulaire de part et d'autre de la colonne vertébrale sur une superficie de 120 cm² environ, en laissant une bande de poils le long du rachis afin de bien séparer les 2 plages. Ils sont toujours laissés au repos pendant au moins quatre heures entre la tonte et l'application du produit. La vitesse de repousse des poils, en particulier au niveau des zones de repousse, a nécessité un rythme de tonte de trois fois par semaine.

Sur les 6 lapins du lot traité, le produit est appliqué 5 jours consécutifs par semaine pendant 6 semaines, au même moment de la journée.

On applique une dose d'huile d'arachide époxydée selon l'invention et tout au long de l'expérimentation elle est de 2 g de produit par animal et par jour, soit, en tenant compte de la densité du produit, 2,2 ml, distribués au moyen d'une seringue de 5 ml sur la plage de droite. Un léger massage à l'aide d'un doigtier en latex de qualité chirurgicale à usage unique permet la pénétration du produit. Le côté gauche ne reçoit pas de produit mais subit un massage identique. Pour éviter le léchage et le grattage des animaux après l'application, ceux-ci sont maintenus ensuit pendant une heure dans des boites à contention individuelles. La pénétration du produit étant totale, il n'est pas procédé à un essuyage ou à un lavage à la sortie de la boite à contention. La peau des animaux reste en permanence à l'air libre.

Les animaux traités et témoins sont pesés chaque semaine et les courbes de croissance moyenne comparatives des deux lots sont établies.

L'aspect général des lapins, leur comportement, leur prise de nourriture et de boisson sont restés normaux pendant toute la durée de l'expérimentation.

Les courbes de croissance des animaux traités (en traits discontinus) et témoins (en traits pleins) sont reportées sur la figure 4. Un des lapins témoins a dû être sacrifié à la fin de la 3e semaine de l'étude car il s'était cassé la colonne vertébrale. Sa disparition n'as pas entraîné de modification de la courbe moyenne de croissance des animaux témoins (cette courbe reste identique du début de l'étude au sacrifice de l'animal que la moyenne pondérale soit faite en incluant ou non le poids de ce lapin).

On voit que la croissance des animaux traités est plus rapide que celle des témoins et l'écart va en augmentant au cours de l'expérimentation.

Des résultats similaires sont obtenus avec une huile de colza peroxydée par transformation biologique à fabile énergie.

Exemple 6

On réalise un essai clinique sur 26 personnes atteintes de diverses maladies. L'essai a été réalisé sur des arthroses du genou et sur un certain nombre de rhumatismes articulaires, tels que périarthrite des hanches, épicondylite, tendinite d'Achille, etc.

Les résultats sont donnés dans le tableau I suivant, pour des applications cutanées d'huile d'arachide transformée biologiquement à faible énergie.

Tableau I

| Affection | Début du traitement | Fin du traitement | Résultats | Effets secondaires |
|---|---|---|---|---|
| gonarthrose | 22.12 | 26.01 | bon résultat | – |
| rhizarthrose bilatérale | 22.11 | 22.12 | échec | – |
| périarthrite de l'épaule droite | 22.11 | 15.12 | amélioration | – |
| épicondylite bilatérale | 26.11 | non revue | échec | – |
| chondromalacie rotuliènne | 26.11 | 30.11 | échec | allergie |
| périarhrite bilatérale des épaules | 29.11 | 10.01 | amélioration | – |
| polyalgies diffuses | 01.12 | 26.01 | bon résultat au 26.12.82 échec au 26.01.83 | – |
| gonarthrose droite | 01.12 | 26.12 | échec | – |
| gonarthrose bilatérale | 01.12 | 31.01 | bon résultat au 20.12.82 et au 31.01.83 | – |
| périarthrite de hanche gauche | 01.12 | 12.01 | résultat moyen | – |
| tendinite du biceps droit | 01.12 | 03.01 | résultat moyen | – |
| épicondylite droite | 03.12 | 20.12 | résultat moyen | – |
| épicondylite bilatérale | 08.12 | 22.12 | résultat moyen | allergie |
| périarthrite de hanche droite | 06.01 | 31.01 | résultat moyen | – |
| arthrose du coude droit | 20.12 | 15.01 | résultat moyen | – |
| douleurs intercostales droites | 07.01 | 26.01 | assez bon résultat | – |
| gonalgies sur gonarthrose | 07.01 | 28.01 | échec | – |
| épicondylite bilatérale | 08.11 | 28.11 | échec | – |
| lombalgies chroniques | 08.11 | 03.12 | amélioration | – |
| tendinite du biceps droit | 08.11 | 24.01 | amélioration | – |
| gonarthrose gauche | 08.11 | 03.01 | amélioration et rechute | – |

## Tableau I (suite)

| Affection | Début du traitement | Fin du traitement | Résultats | Effets secondaires |
|---|---|---|---|---|
| coxarthrose droite + | | | | trouve que cela |
| gonarthrose droite | 10.11 | 10.01 | amélioration et rechute | sent mauvais |
| tendinite d'Achille gauche | 15.11 | 10.12 | échec | – |
| gonalgies gauches | 01.12 | 30.12 | échec | – |
| arthrose tibio-tarsienne | 05.12 | 08.01 | échec | – |
| gonarthrose gauche | 08.11 | 20.11 | bon résultat | – |
| gonalgies bilatérales | 12.12 | 08.02 | succès puis échec | – |
| gonarthrose bilatérale | 15.11 | 20.12 | amélioration | – |

Exemple 7

On effectue des expérimentations sur des animaux, en particulier les équidés, qui reçoivent des applications cutanées d'huile d'arachide transformée biologiquement à faible énergie.

On obtient les résultats suivants, consignés dans le tableau II ci-dessous.

Les affections traitées sont: mollettes, crevasses, oedèmes, raideurs, douleurs, dermatose.

## Tableau II

| Nom | Indications | Résultats |
|---|---|---|
| FL | | |
| OB | crevasses | succès |
| FL | | |
| MIS | crevasses | succès |
| AV | crevasses | succès |
| SAN | crevasses | succès |
| BEL | crevasses | succès |
| ORE | crevasses | échec |
| LIO | crevasses | succès |
| VAL | crevasses | succès |
| MAT | crevasses | moyen |
| LI | | |
| GUI | crevasses | succès |
| OV | crevasses | succès |
| BEL | crevasses | bon résultat |
| ADU | crevasses | succès |
| NOM | crevasses | moyen |
| ME | crevasses | succès |
| MER | crevasses | échec |
| SH | crevasses | succès |
| TER | crevasses | succès |
| FL | œdèmes | moyen |
| AV | œdèmes | succès |
| JA | œdèmes | moyen |
| KIL | œdèmes | succès |
| LI | œdèmes | succès |
| GUI | œdèmes | bon résultat |
| OV | œdèmes | moyen |
| STA | œdèmes | succès |
| KOR | œdèmes | bon resultat |
| STA | molettes | succès puis récidive puis succès |
| KO | molettes | succès |
| RE | molettes | succès |
| FL | dermatoses | moyen |

## Tableau II (suite)

| Nom | Indications | Résultats |
|---|---|---|
| OB | dermatoses | succès |
| LY | dermatoses | succès |
| MEL | dermatoses | moyen |
| DI | dermatoses | succès |
| MER | | |
| NOT | dermatoses | échec |
| OB | douleurs-raideurs | moyen |
| FL | | |
| MIS | douleurs-raideurs | très bon résultat |
| AV | douleurs-raideurs | très bon résultat |
| SAN | douleurs-raideurs | très bon résultat |
| VAL | douleurs-raideurs | très bon résultat |
| MAI | | |
| LAI | douleurs-raideurs | moyen |
| GUI | | |
| OV | douleurs-raideurs | échec |
| STA | douleurs-raideurs | moyen |
| KOR | douleurs-raideurs | moyen |
| SHO | douleurs-raideurs | très bon résultat |
| RED | douleurs-raideurs | très bon résultat |
| TER | douleurs-raideurs | moyen |

L'action d'huile d'arachide peroxydée biologiquement à faible énergie est souvent rapide, voire spectaculaire: appliquée avant la compétition, elle a permis d'éviter dans une majorité de cas toutes les raideurs ou oedèmes, habituels chez certains sujets. Appliquée après la compétition, elle a permis une récupération musculaire nette.

Elle a eu un effet très bénéfique dans 3 cas de mollettes: action plus rapide et plus efficace qu'avec les moyens traditionnels.

Appliquée au débourrage chez les jeunes sujets, elle a permis de résoudre les problèmes d'irritation au passage de sangle et la disparition des boutons survenus lors du travail au Mors (péri-buccal).

L'action la plus spectaculaire concerne les crevasses. L'huile a permis une cicatrisation nette, rapide, avec un produit sans effet rebond: cette action n'a jamais été obtenue dans ces conditions avec les produits habituellement utilisés. L'action a été spectaculaire lorsque les crevasses ont été traitées à leur début.

Utilisée à l'entraînement, elle a permis une meilleure souplesse des zones tendineuses et des membres. Chez les sujets sensibles au boulet, on a constaté un meilleur aspect après le travail.

L'huile traitée selon l'invention est donc antalgique, cicatrisante, et a un effet très bénéfique sur les dermatoses et les dermites de contact.

Exemple 8

On effectue d'autres essais cliniques sur des malades. On applique l'huile peroxydée biologiquement à faible énergie par massages chaque soir avant le coucher. On effectue deux applications suivies de massage. Le traitement dure entre 10 et 15 jours.

Les résultats sont consignés dans le tableau III suivant.

Tableau III

| Nom | Indications | Résultats |
|-----|-------------|-----------|
| LE BR | escarres | bon |
| CARD | escarres | moyen |
| JON | escarres | exceptionnel très bon |
| PRAD | hémorroïdes | très bon |
| LE D | escarres | bon |
| BOL | escarres | amélioration |
| CY | escarres | exceptionnel |
| DEC | escarres | moyen |
| LAU | escarres | bon |
| PLA | escarres | bon |
| ROD | escarres | bon |
| CRI | abcès du sein | bon |
| FRE | abcès du sein | moyen |
| GLA | ulcère variqueux | échec |
| LE GE | escarres | exceptionnel |
| HEL | escarres | échec |
| MIN | escarres | bon |
| LE CA | fistules périnéales | moyen |
| DIR | ulcère variqueux | très bon |
| LOU | hémorroïdes | exceptionnel |
| STE | hémorroïdes | bon résultat |
| GUEL | ulcère variqueux | moyen |
| LE PA | hémorroïdes | échec |
| JAM | cicatrices d'épisiotomies | bon résultat |
| DOU | fistules périnéales | moyen |
| MART | fistules périnéales | bon |
| BOS | abcès du sein | bon |
| CRON | hémorroïdes | exceptionnel |
| LER | cicatrices d'épisiotomies | bon |
| BRAN | cicatrices d'épisiotomies | exceptionnel |

Exemple 9

On traite des animaux, tels qu'ovins et bovins par de l'huile d'arachide peroxydée biologiquement à faible énergie.

L'huile peroxydée est appliquée directement sur la peau de certains sujets présentant des lésions cutanées spécifiques, soit à l'aide d'un pinceau, soit à la main.

Essais

a) Maladie des oreilles: apparition de nombreux boutons, secs, préludant à un gonflement important de l'oreille qui finit par s'affaisser et pourrit.

b) Dermatophilose avec épilation: formations croûteuses plus ou moins étendues sur la peau et parfois confondues avec celles de la gale mais exemptes de psoroptes et de prurit caractéristiques de cette maladie, en très forte extension depuis quelques années et appelée aussi maladie des laines jaunes.

c) Gale psoroptique, due à l'infestation d'acariens, les psoroptes. La peau elle-même est très indurée, devient croûteuse. Prurit intense – contagiosité extrême. Les résultats sont les suivants:
– la destruction des causes apparentes ou cachées;
– la rapidité étonnante de cicatrisation (couche papillaire du derme);
– la régénération immédiate du derme (couche réticulaire).

Les résultats les plus spectaculaires sont ceux concernant la Gale psoroptique où, 36 heures après l'application manuelle de l'huile, le derme retrouve sa souplesse et les symptômes de la maladie ont localement disparus.

Exemple 10 détermination de la dose létale 50 D'une part, on administre à un lot n° 1 de souris mâles des doses de 0,5 ml d'huile d'arachide peroxydée par transformation à faible énergie par 20 g de poids corporel, soit 25 ml/kg.

D'autre part, on administre à un lot n° 2 de souris mâles la même huile à des doses de 1 ml par 20 g soit 50 ml/kg, en deux fois, à 4 heures d'intervalle.

Des souris témoins ont reçu dans les mêmes conditions de l'huile d'arachide non peroxydée, pure, et constituent les lots 3 et 4.

Les animaux ont été gardés à l'animalerie pendant une période d'acclimatation de 7 jours avant l'essai et ont reçu de l'aliment complet pour rats et souris et de l'eau de ville distribuée en biberons, ad libitum. Ils ont été mis à jeun 18 heures avant la première série de gavages. Après l'administration (ou les 2 administrations) de produit, les animaux ont été gardés en observation pendant une période de 14 jours. Les résultats obtenus sont rassemblés dans le tableau IV.

Tableau IV

| Produit administré | Lot n° | Dose en ml/kg de poids corporel | Mortalité |
|--------------------|--------|----------------------------------|-----------|
| Huile peroxydée | 1 | 25 | 1/10 |
| Huile peroxydée | 2 | 50 (2×25,4 h. interv.) | |
| Huile d'arachide pure | 3 | 25 | 0/10 |
| Huile d'arachide pure | 4 | 50 (2×25,4 h. interv.) | 0/10 |

Une souris du lot n° 1 (1 fois 25 ml/kg d'huile peroxydée) est morte 4 jours après l'administration du produit sans avoir présenté de symptômes préalables. La mortalité a été nulle dans tous les autres lots.

Chez les animaux du lot n° 2, ayant reçu 2 fois 25 ml/kg d'huile peroxydée, on observait le lendemain de l'ingestion un aspect huileux et hérissé des poils du museau et de l'arrière train. On notait également une légère diarrhée. La mobilité de tous les animaux était normale. Après 48 heures, on notait une amélioration de l'aspect des animaux et un retour complet à la normale le troisième jour.

Les animaux de tous les autres lots ont toujours présenté un aspect et un comportement normaux.

Tous les animaux survivants ont été sacrifiés après 14 jours d'observation, par anesthésie dépassée au pentobarbital sodique administré par voie intrapéritonéale. Hormis un aspect plus ou moins congestif du poumon chez tous les animaux, traités et témoins, tous les organes présentaient un aspect normal à l'examen macroscopique.

En conclusion, l'administration par voie orale d'huile peroxydée est bien tolérée par la souris à des doses très élevées.

Bien entendu, on peut réaliser des mélanges du principe actif constitué par le corps gras peroxydé par transformation biologique à faible énergie avec des supports, des sels acceptables pharmacologiquement, avec d'autres principes actifs, ou protéines végétales, animales, huiles essentielles.

Selon l'invention, il est possible de traiter après filtrage, des corps gras alimentaires usagés, qui sont alors régénérés et avantageusement utilisés industriellement comme produits de substitution à des matières onéreuses par exemple utilisées dans l'industrie des peintures, vernis, colorants, enduits, cirages, produits pour tannerie et mégisserie. Notamment, par le traitement des peaux par les corps gras selon l'invention, on obtient une hydratation et une nourriture exceptionnelles des peaux, ce qui permet un gain de superficie des peaux par étirage.

En outre, les corps gras ayant subi la transformation à faible énergie selon l'invention amplifient le pouvoir des principes actifs avec lesquels ils sont mis en synergie, permettant ainsi de diminuer les quantités à employer au niveau industriel, tout en conservant au minimum les effets identiques.

## Revendications

1. Procédé de peroxydation de corps gras tel qu'on introduit progressivement et en continu un mélange gazeux oxygéné dans le corps gras, de préférence liquide, en faisant varier par paliers irréguliers la température du corps gras, et en maintenant la température sensiblement constante pendant des phases variables de durées déterminées, caractérisé par le fait qu'on effectue alternativement des montées et descentes en température, les durées des phases peuvent être considérées comme des cycles $(n \times 7 \pm 1)h$, n étant un nombre entier.

2. Procédé selon la revendication 1, caractérisé par le fait que les durées des phases de traitement sont égales à environ 34, 41 ou 49 h.

3. Procédé selon l'une quelconque des revendications 1 et 2, caractérisé par le fait que les phases de traitement peuvent être séparées par au moins une phase de repos pendant laquelle la température est égale à la température ambiante ou voisine de 0°C.

4. Procédé selon la revendication 3, caractérisé par le fait que la durée de la phase de repos est un multiple de 7 h, de préférence est égale à 147 heures environ.

5. Procédé selon l'une quelconque des revendications 1 à 4 caractérisé par le fait qu'on récupère et conserve le produit obtenu après la phase de traitement pendant laquelle le mélange réactionnel subit le changement de couleur.

6. Procédé selon l'une quelconque des revendications 1 à 5, caractérisé par le fait qu'après une première étape de traitement et avant une deuxième étape de traitement, on effectue une phase de «pont modulant» pendant laquelle la température est maintenue constante, et de durée sensiblement égale à un multiple de 7 h, de préférence égale à 42 h ou 70 h.

7. Procédé selon l'une quelconque des revendications 1 à 6, caractérisé par le fait qu'on traite un mélange d'un corps gras peroxydé préalablement traité avec un produit choisi dans le groupe des extraits et embryons végétaux, des protéines végétales et animales, des organites cellulaires, des vitamines, des oligo-éléments, des minéraux, des métaux, des solutions aqueuses, des macérats, des huiles essentielles.

8. Procédé selon l'une quelconque des revendications 1 à 7, caractérisé par le fait qu'on accélère les réactions par induction.

9. Application de corps gras peroxydé selon les revendications 1 à 8, comme catalyseur bio-énergétique et activateur des échanges chimiques, gazeux ou bio-électriques, notamment dans le traitement des cuirs et peaux, du bois, des produits de finition (peinture, vernis, colorants, cirages), comme produits diététiques, cosmétiques, agro-alimentaires et nouveaux carburants.

10. Médicament à action polyvalente (antalgique, facteur de croissance, cicatrisant, anti-inflammatoire, bactériostatique), caractérisé par le fait qu'il comporte un corps gras peroxydé, selon l'une quelconque des revendications 1 à 8.

## Patentansprüche

1. Verfahren um fette Körper zu peroxidieren, wobei man kontinuierlich und fortschreitend eine oxidierende Gasmischung in den fetten Körper, vorzugsweise eine Flüssigkeit einleitet, indem man in unregelmässigen Etappen die Temperatur des fetten Körpers variiert und indem man die Temperatur während variabler Phasen vorbestimmter Dauer möglichst konstant hält, dadurch gekennzeichnet, dass man abwechselnd Temperaturanstiege und Absenkungen vornimmt, wobei die Dauern der Phasen als Zyklen angenommen werden können (n*7±1) Stunden, wobei n eine ganze Zahl ist.

2. Verfahren nach Anspruch 1, dadurch gekennzeichnet, dass die Dauer der Behandlungsphasen gleich ist etwa 34, 41 oder 49 Stunden.

3. Verfahren nach einem der Ansprüche 1 oder 2, dadurch gekennzeichnet, dass die Behandlungsphasen durch mindestens eine Ruhephase getrennt sein können, während der die Temperatur gleich ist der Umgebungstemperatur oder im Bereich von 0°C liegt.

4. Verfahren nach Anspruch 3, dadurch gekennzeichnet, dass die Dauer der Ruhephase ein Vielfaches von 7 Stunden, vorzugsweise etwa 147 Stunden ist.

5. Verfahren nach einem der Ansprüche 1 bis 4, dadurch gekennzeichnet, dass man das erhaltene Produkt nach der Phase der Behandlung während der die Reaktionsmischung der Farbenänderung unterliegt, entnimmt und aufbewahrt.

6. Verfahren nach einem der Ansprüche 1 bis 5, dadurch gekennzeichnet, dass nach einer ersten Behandlungsstufe und vor einer zweiten Behandlungsstufe eine «Modulationsüberbrückungsphase» durchgeführt wird, während der die Temperatur konstant gehalten wird und deren Dauer möglichst genau ein Vielfaches von 7 Stunden ist, vorzugsweise gleich 42 oder 70 Stunden.

7. Verfahren nach einem der Ansprüche 1 bis 6, dadurch gekennzeichnet, dass man eine Mischung eines fetten Körpers behandelt, der vorher mit einem Produkt aus der Gruppe der pflanzlichen Extrakte und Keime, der pflanzlichen und tierischen Proteine, der Zellorganismen, der Vitamine, der Spurenelemente, der Minerale, der Metalle, der wässrigen Lösungen, der Mazerate und der essentiellen Öle behandelt worden ist.

8. Verfahren nach einem der Ansprüche 1 bis 7, dadurch gekennzeichnet, dass man die Reaktionen durch Induktion beschleunigt.

9. Anwendung des gemäss den Ansprüchen 1 bis 8 peroxidierten fetten Körpers als bioenergetischer Katalysator und Aktivator des chemischen Austausches, gasförmig oder bioelektrisch, insbesondere bei der Behandlung von Leder und Häuten, Holz, Endbehandlungsprodukten (Farben, Anstrichen, Wachsen, Firnissen) als diätetische, kosmetische, Agro-Versorgungs-Produkte und als neue Brennstoffe.

10. Medikament mit mehrfacher Wirkung (schmerzstillend, Wachstumsfaktor, wundheilend, entzündungshemmend, bakterizid), dadurch gekennzeichnet, dass es einen nach einem der Ansprüche 1 bis 8 peroxidierten fetten Körper enthält.

## Claims

1. Process for the peroxidation of fatty substances such that an oxygenated gaseous mixture is progressively and continuously introduced into the fatty substance, preferably liquid, by varying the temperature of the fatty substance in irregular stages, and by keeping the temperature substantially constant during variable phases of predetermined durations, characterized by the fact that rises and falls of temperature are alternated, the durations of the phases can be considered as cycles ($n \times 7 \pm 1$) hr, n being an integer.

2. Process according to claim 1, characterized by the fact that the durations of the treatment phases are equal to about 34, 41 or 49 hrs.

3. Process according to any one of claims 1 and 2, characterized by the fact that the treatment phases can be separated by at least a phase of rest during which the temperature is equal to room temperature or close to 0°C.

4. Process according to claim 3, characterized by the fact that the duration of the phase of rest is a multiple of 7 hrs, preferably is equal to about 147 hours.

5. Process according to any one of claims 1 to 4, characterized by the fact that the resulting product is recovered and preserved after the phase of treatment during which the reaction mixture undergoes a change of color.

6. Process according to any one of claims 1 to 5, characterized by the fact that after a first step of treatment and before a second step of treatment, a phase of «modulating bridge» during which the temperature is kept constant is effected, and having a duration substantially equal to a multiple of 7 hrs, preferably equal to 42 hrs or 70 hrs.

7. Process according to any one of claims 1 to 6, characterized by the fact that a mixture of previously treated peroxidized fatty substance is treated with a product selected from the group of vegetable extracts and embryos, vegetable and animal proteins, cellular organoids, vitamins, trace elements, minerals, metals, aqueous solutions, macerates, essential oils.

8. Process according to any one of claims 1 to 7, characterized by the fact that the reactions are accelerated by induction.

9. Application of the peroxidized fatty substance according to claims 1 to 8, as bio-energizing catalyst and activator of chemical, gaseous or bioelectrical exchanges, in particular in the treatment of leather and skins, wood, finishing products (paint, varnishes, dyes, polishes), as dietetic, cosmetic, agroalimentary products and novel fuels.

10. Drug having a polyvalent action (antalgic, growth factor, cicatrizing, anti-inflammatory, bacteriostatic), characterized by the fact that it comprises a peroxidized fatty substance according to any one of claims 1 to 8.

FIG. 1

FIG. 2

FIG. 3

0117962

FIG. 4